# EUROPEAN PATENT SPECIFICATION

(11) **EP 1 453 561 B1**
(45) Date of publication and mention of the grant of the patent: **31.08.2016**
(21) Application number: 02784781.3
(22) Date of filing: 11.12.2002
(51) Int. Cl.: A61M 5/32, B65D 83/10

(54) **NEEDLE CLOSURE SYSTEM REMOVAL DEVICE**
VORRICHTUNG ZUR ENTFERNUNG EINES NADEL-VERSCHLUSSSYSTEMS
DISPOSITIF POUR L'ENLÈVEMENT D'UN SYSTÈME D'OBTURATION D'AIGUILLE

(30) Priority: 13.12.2001 US 341328 P
(43) Date of publication of application: 08.09.2004
(60) Divisional application: 13182835.2
(73) Proprietor: Becton Dickinson and Company, Franklin Lakes, NJ 07417-1880 (US)
(72) Inventor: BARRELLE, Laurent, F-38250 Saint Nizier du Moucherotte (FR); PEROT, Frederic, F-38000 Grenoble (FR); VEDRINE, Lionel, F-38400 Saint-Martin D'heres (FR)
(74) Representative: dompatent von Kreisler Selting Werner - Partnerschaft von Patent- und Rechtsanwälten mbB
(86) International application number: PCT/US2002/039607
(87) International publication number: WO 2003/051423

(56) References cited:
- US-A- 4 636 201
- US-A- 4 986 817
- US-A- 5 078 696
- US-A- 5 564 565
- US-A- 5 718 689

## Description

### TECHNICAL FIELD

The subject invention relates generally to a safety device used to remove a shield from a medical assembly. More particularly, the subject invention relates to a safety device for protecting a user of the medical assembly from being pricked by a needle or other sharp device when removing a protective shield.

### BACKGROUND OF THE INVENTION

This application claims priority to United States Provisional Patent Application No. 60/341,328 filed December 13, 2001. A needle assembly is typically attached to a hypodermic syringe to either inject a substance into the skin of a patient or to withdraw blood from the patient. Other types of devices having exposed sharpened tips also staked or attached to a medical instrument are used to administer a medical procedure. A typical needle assembly includes a needle cannula having a sharpened tip capable of piercing the skin of a patient. The needle cannula or other device having a sharpened tip is commonly covered by a shield to maintain the cannula in a clean if not sterile condition and to prevent unintentional needle pricks of the user. Alternatively, a user may need to be protected from contact with an assembly having an exposed tip or connection feature that is not sharp. Contact with the tip may cause injury if the contents of the assembly are toxic, or contaminate the contents with a virus or bacteria. Therefore, an assembly with a blunt tip may also require a shield to maintain the tip in a clean or sterile condition. Prior to using the medical assembly to perform the desired medical procedure, the shield must be removed to expose the needle cannula or other sharpened or exposed tip or connection device.

Medical providers may accidentally be pricked by the needle while removing the shield from the assembly. Therefore, various devices have been introduced to shield the medical provider from the needle while removing the shield. Two such devices are disclosed in United States Patent Nos. 5,078,696 and 4,986,817. The devices disclosed in these patents grip the shield with friction forces resultant from inserting the shield into the tubular member. However, because various sizes and configurations of shields have been introduced to the market place, the devices may not always provide an adequate friction force to withdraw the shield from the assembly due to a poor fit between the device and the shield.

The devices disclosed in these patents are also used to reinsert the needle cannula into the shield after the desired medical procedure has been administered to the patient. This is known to be an undesirable practice because a needle cannula can become contaminated upon withdrawal from a needle shield if it comes into contact with the surface of the device that has been contaminated by a used needle assembly while reinserting the used needle assembly into the needle shield.

It would be desirable to provide a shield removal device capable of removing various sizes and configurations of shields. Further, it would be desirable to provide a device that permanently clasps the shield to prevent the shield removal device from being reused. Still further, it would be desirable to provide a device that allows the shield to be separated from the device after removing the shield from the medical instrument to enable repeated use of the device.

### SUMMARY OF THE INVENTION

A shield removal device for removing a shield from a sharp tipped medical assembly is disclosed. A tubular member includes a lower end and an upper end that is dimensioned to receive the shield. The tubular member includes an inner wall having at least one gripping element with a notch providing a flex point upon the gripping element that enables the gripping element to flex radially outwardly. By flexing radially outwardly, the tubular member receives the shield, and permanently clasps the shield allowing the sharp tipped medical assembly to be withdrawn from the shield.

The disclosed shield removal device solves the problems associated with the prior art devices with the gripping elements that expand outwardly to receive various dimensioned shields and snap back to clasp a lip or edge of the shield. Further, the gripping elements may be configured to permanently clasp onto the shield inserted into the tubular member to prevent the inventive shield removal device from being reused.

### BRIEF DESCRIPTION OF THE DRAWINGS

Other advantages of the present invention will be readily appreciated as the same becomes better understood by reference to the following detailed description when considered in connection with the accompanying drawings wherein:
Figures 1 - 4 show various shields or covers presently in use;
Figure 5 show a perspective view of a first embodiment of the shield removal device of the present invention;
Figure 6 shows a sectional view of the gripping element through line 6-6 of Figure 5;
Figure 7 shows a perspective view of an alternate embodiment of the shield removal device;
Figure 8 - 10 shows perspective views of a shield being removed by the alternate embodiment of the shield removal device;
Figure 11A and 11B shows a sectional view of the alternate embodiment of the shield removal device;
Figure 12A - 12C show sectional views of the alternate shield removal device clasping the lip of a shield;
Figures 13 - 15 show sectional views of the alternate shield removal device clasping the lip of differently configured shields;
Figure 16 - 17 show perspective views of an alternate embodiment of the shield removal device;
Figure 18A - 18C show a sectional view of alternate gripping elements of the alternate shield removal device through line 18-18 of Figure 17; and
Figure 19 shows a further alternate embodiment of the shield removal device.

### DETAILED DESCRIPTION OF THE INVENTION

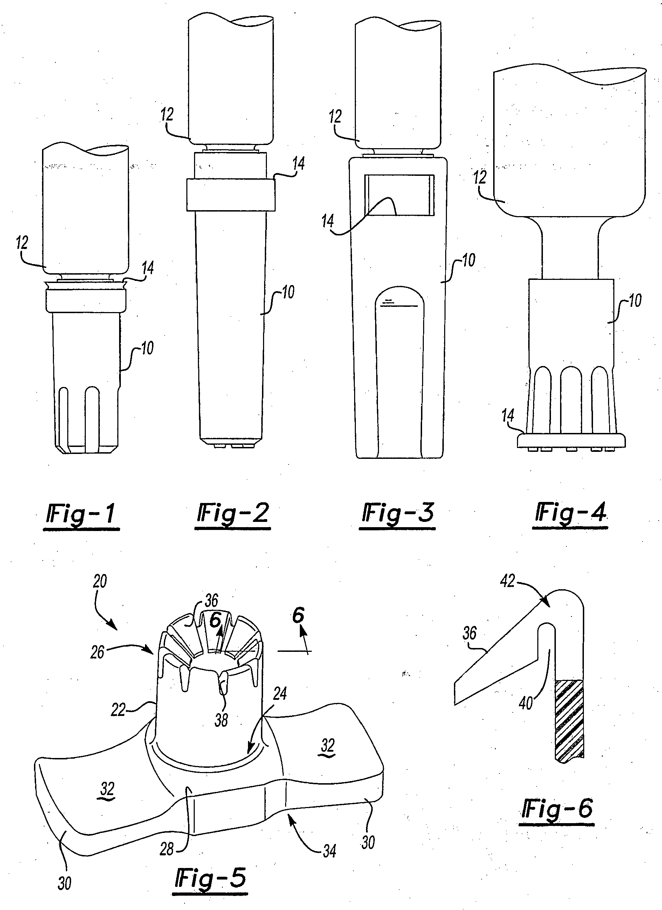

Referring to Figures 1 through 4, various types of shields 10 that shield an exposed tip of a medical assembly, such as, for example, a needle cannula or other skin piercing device or connecting tip are shown. Figure 4 represents a shield that covers a luer tip to which a hypodermic needle is attached after the shield is removed. Different types of medical devices have different dimensions and configurations. Therefore, the shields 10 used to shield the medical devices have different dimensions. These shields 10 also have different removal forces to overcome to expose the underlying needle, tip or connection to administer the desired medical procedure. Common to all of the shields 10 is a lip or ridge 12 disposed somewhere upon the shield that can be clasped to assist withdrawing the shield 10 from the assembly.

A first embodiment of an inventive shield removal device is generally shown at 20 of Figure 5. The device 20 includes a tubular member 22 having a lower end 24 and an upper end 26. The upper end 26 is dimensioned to receive shields 10 having a variety of dimensions and configurations. A base 28 includes opposing arms 30 extending radially outwardly from the tubular member 22. Each arm 30 may include an upper surface 32 having a generally arcuate shape providing a contoured depression surface for stabilizing the shielding device. The base 28 includes a generally planar lower surface 34 providing an abutment against, for example, a flat surface (not shown) enabling the device 20 to be stabilized when the upper surface 32 of the opposing arms 30 is depressed.

The upper end 26 of the tubular member 22 includes a plurality of gripping elements 36 extending inwardly of the tubular member 22 for clasping the shield 10. Each gripping element 36 is separated by a gap 38 disposed longitudinally in the tubular member 22. Each gap 38 allows each gripping element 36 to flex radially outwardly when the tubular member 22 receives the shield 10. Referring to Figure 6, the gripping element 36 is shown in a partial sectional view along line 6-6 of Figure 5. Each gripping element 36 includes a notch 40 that provides a flex point 42 enabling the gripping element 36 additional flexible movement radially outwardly to receive the shield 10 and clasp on to the lip or ridge 14 of the shield 10. The notch 40 opens generally downwardly towards the lower end 24 of the tubular member 22.

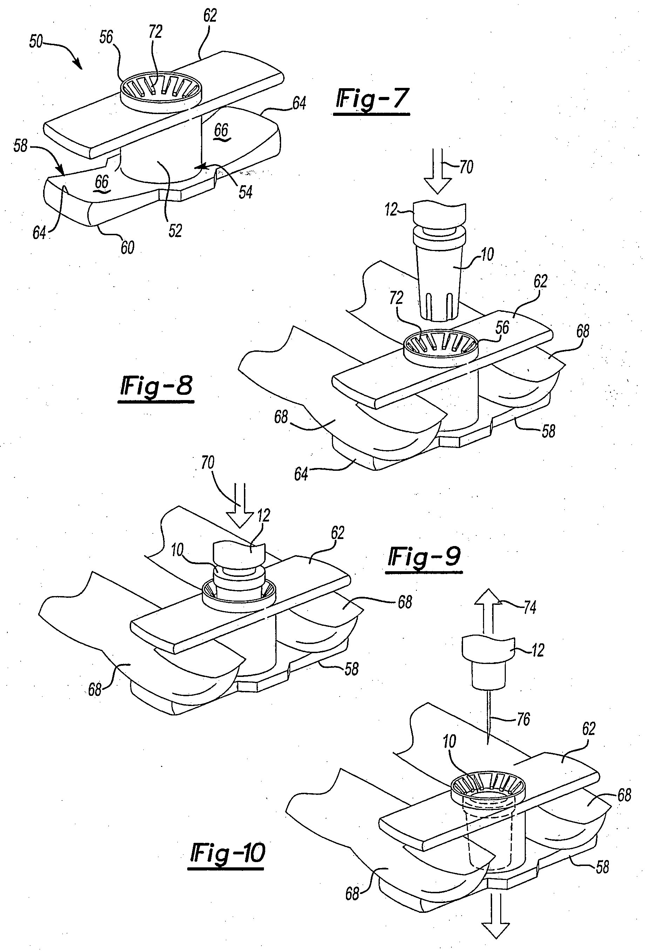

An alternative embodiment of the inventive shield removal device is generally shown at 50 of Figure 7. The alternative shield removal device 50 includes a tubular member 52 having a lower end 54 and an upper end 56 that is dimensioned to receive the shield 10. A base 58 extends outwardly from the lower end 54 of the tubular member 52 and has a generally planar lower surface 60. A guard 62 extends outwardly from the tubular member 52 and is spaced from the base 58 providing a gap between the base 58 and the guard 62. The base 58 includes opposing arms 64 each having an upper surface 66. Each upper surface 66 may include a generally arcuate shape providing a contoured depression surface for stabilizing the alternate needle shield device 50 by depressing downwardly upon the upper surface 66.

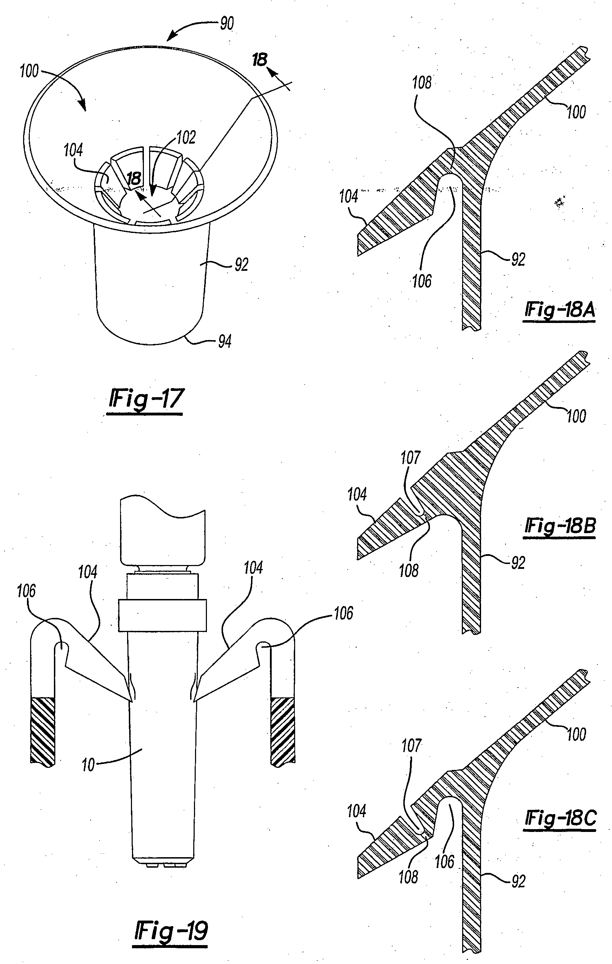

Figures 8, 9 and 10 show the method by which the shield 10 is removed from the needle assembly 12 using the inventive alternate shield removal device 50. A medical provider's fingers 68 are inserted into the gap disposed between the guard 62 and the base 58. The medical provider depresses the device 50 downwardly onto a flat surface (not shown) in a stable manner, which is assisted by the generally arcuate upper surface 66. The needle assembly 12 is inserted downwardly in a direction of arrow 70 shown in Figure 8 and 9 into the upper end 56 of the tubular member 52. Gripping elements 72 extending inwardly of said tubular member 52 and expand radially outwardly as described above to receive the shield 10 and engage a ridge or upper lip 14 of the shield 10 permanently securing the shield 10 to the device 50 as shown in Figure 10. Alternatively, as shown in. Figure 19, each gripping element 72 may include a sharpened tip capable of penetrating the side of the shield 10 to clasp the shield 10 for removal. Withdrawing the needle assembly 12 from the shield 10 in the direction of arrow 74 as shown in Figure 10 exposes a needle 76 or other tip or connection feature. The medical provider can now provide the desired medical procedure having safely removed the needle cannula 76 from the shield 10. Subsequent to administering the medical procedure, the needle can either be reinserted into the shield 10 in the combination of the needle assembly 12 and the needle shield removal device 50 disposed of, or, preferably, the needle assembly 12 and the needle shield 10 and device 50 can be disposed of separately.

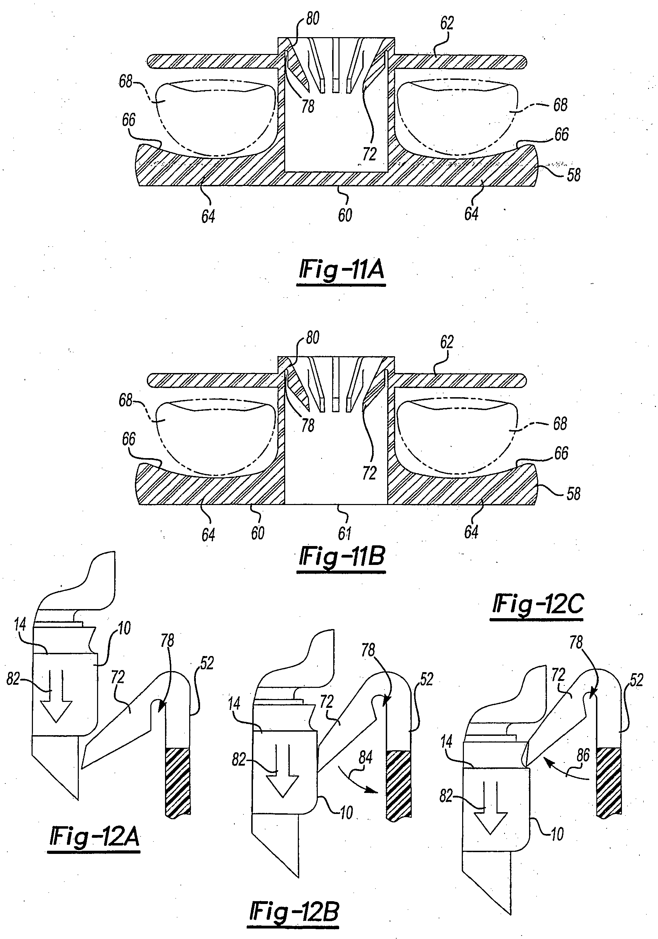

Figure 11A shows a sectional view of the alternate needle shield removal device 50 and best represents the gripping elements 72 that extend inwardly of the tubular member 52. Each gripping element 72 is spaced radially around the tubular member 52. A notch 78 is disposed in each gripping element 72 providing a flex point 80 in the gripping element 72. The notch 78 opens generally downwardly towards the lower end 54 of the tubular member 52. The flex point 80 enables the gripping element 72 to flex radially outwardly to receive the needle shield 10 and to permanently clasp the needle shield 10 allowing the needle assembly 12 to be withdrawn from the shield 10. An alternate embodiment is shown in Figure 11B having an opening 61 disposed below the tubular member 52, which allows the shield 10 to be ejected from the tubular member 52. This would enable the device 50 to be reused as needed.

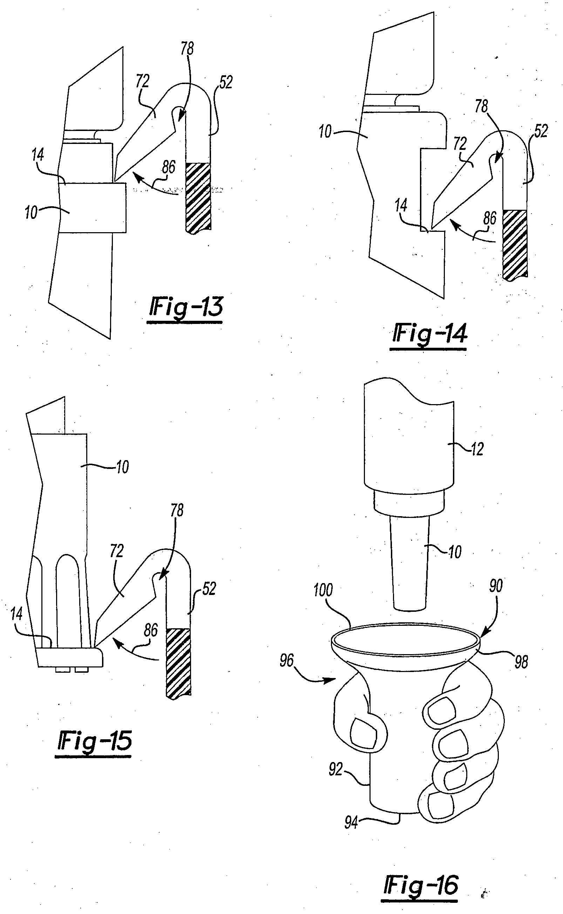

Figures 12A through 12C show the flex action of the gripping element 72 to receive the shield 10 and to clasp permanently the ridge or lip 14 of the needle shield 10. Figure 12A shows the shield 10 initially being inserted into the tubular member 52 in the direction of arrow 82. Figure 12B shows the gripping element 72 flexing radially outwardly in the direction of arrow 84 to receive the shield 10. Figure 12C shows the gripping element 72 flexing inwardly in the direction of arrow 86 toward original position locking onto the ridge or lip 14 of the shield 10 permanently retaining the shield 10 in the tubular member 52. Figures 13 through 15 show the gripping element 72 permanently clasping differently configured shields 10 at the ridge or lip 14 by flexing inwardly toward original position in the direction of arrow 86. Figure 14 shows the gripping element 72 engaging a window commonly disposed within the shield 10. The window may also commonly be formed in a rigid sheath that covers a rubber or elastomeric shield.

A further alternate embodiment of the shield removal device is generally shown at 90 in Figures 16, 17 and 18A -C. A tubular member 92 includes a lower end 94 and an upper end 96 dimensioned to receive a shield 10. A generally conical or frustoconical member 98 includes a large diameter opening 100 and a small diameter opening 102 integrally formed with the upper end 96 of the tubular member 92.

The tubular member 92 includes a plurality of gripping elements 104 extending inwardly of the tubular member 92. Each gripping element 104 includes at least one notch 106 as represented in cross sectional view shown in Figure 18A-C pointing downwardly toward said lower end 94 of said tubular member 92. Alternately, a second notch 107 pointing generally upwardly provides additional flex to each gripping element 104 enabling the gripping element 104 to flex to permanently clasp the shield 10 as shown in Figure 18B and 18C. However, any shape that forms a flex point 108 in the gripping element 104 will suffice. Preferably, the gripping elements 104 are spaced circumferentially around the tubular member 92.

The embodiments of the shield removal device 20, 50, 90 of this invention may be integrally molded from any suitable polymer, including polyethylene, polypropylene, polycarbonate, etc. The embodiments of the shield removal device 20, 50, 90 are also relatively simple in design and therefore inexpensive. Thus, the device of this invention may be preassembled onto a syringe or medical cartridge to minimize and ease manipulation by a health care worker or patient.

The invention has been described in an illustrative manner, and it is to be understood that the terminology which has been used is intended to be in the nature of words of description rather than of limitation.

Obviously, many modifications and variations of the present invention are possible in light of the above teachings. It is, therefore, to be understood that within the scope of the appended claims, wherein reference numerals are merely for convenience and are not to be in any way limiting, the invention may be practiced otherwise than as specifically described.

## Claims

1. A shield removal device for removing a shield from an assembly comprising:
a tubular member (22, 52, 92) having a lower end (24, 54, 94) and an upper end (26, 56, 96) dimensioned to receive a shield (10);
the tubular member (22, 52, 92) includes an inner wall having at least one gripping element (36, 72, 104) extending inwardly therefrom, wherein said at least one gripping element (36, 72, 104) is adapted to flex radially outwardly to receive the shield (10);
**characterized in that**
said at least one gripping element (36, 72, 104) is further adapted to flex inwardly to lock onto a ridge or lip (12) of said shield (10) when said shield (10) is inserted into said tubular member (22, 52, 92);
the shield removal device further comprises
a base (28, 58) extending outwardly from said lower end (24, 54, 94) of said tubular member (22, 52, 92) having a generally planer lower surface (34, 60), and
a guard (62) extending outwardly from said tubular member (22, 52, 92) spaced from said base (28, 58) thereby providing a gap (38) between said base and said guard;
said base (28, 58) including opposing arms (30, 64) each having an upper surface (32, 66) with a generally arcuate shape providing a contoured depression surface for stabilizing said shield removal device.

2. A device as set forth in claim 1 wherein said tubular member (22, 52, 92) includes a plurality of gripping elements (36, 72, 104) extending inwardly of said tubular member (22, 52, 92) for clasping the shield (10).

3. A device as set forth in claim 2 wherein each of said gripping elements (36, 72, 104) include a notch (40, 78, 106) enabling said gripping elements (36, 72, 104) to flex radially outwardly enabling said device to clasp shields (10) having different dimensions.

4. A device as set forth in claim 2 wherein each said gripping element (36, 72, 104) is separated by a notch (40, 78, 106) disposed in said tubular member (22, 52, 92) allowing each said gripping element to flex radially outwardly enabling said device to clasp shields (10) having different dimensions.

5. A device as set forth in claim 1 wherein said base (28, 58) includes a stabilizing member projecting radially outwardly at said tubular member (22, 52, 92) in a generally perpendicular orientation to said opposing arms (30, 64).

6. A shield removal device for removing a shield from an assembly comprising:
a tubular member (22, 52, 92) having a lower end (24, 54, 94) and an upper end (26, 56, 96) dimensioned to receive a shield (10);
the shield removal device further comprises a generally conical member having a large diameter opening (100) and a small diameter opening (102) integrally formed with said upper end (26, 56, 96) of said tubular member (22, 52, 92);
wherein said tubular member (52) includes a plurality of gripping elements (36, 72, 104) extending inwardly of said tubular member (52), each of said plurality of gripping elements having at least one notch (40, 78, 106) providing a flex point (42, 80, 108) upon said gripping elements enabling said gripping elements to flex radially outwardly to receive the shield (10);
**characterized in that**
said plurality of gripping elements (36, 72, 104) are adapted to flex inwardly to lock onto a ridge or lip (12) of said shield (10) when said shield (10) is inserted into said tubular member (22, 52, 92).

7. A shield removal device as set forth in claim 6 wherein said plurality of gripping elements (36, 72, 104) are spaced circumferentially around said tubular member (52).

8. A shield removal device as set forth in claim 6 wherein each said notch (40, 78, 106) opens generally towards said lower end (24, 54, 94) of said tubular member (52).

9. A shield removal device as set forth in claim 6 wherein said notch (40, 78, 106) opens generally towards said upper end (26, 56, 96) of said tubular or conical member.

10. A shield removal device as set forth in claim 6 wherein said gripping elements (36, 72, 104) include two notches (106, 107) opening in generally opposed directions.

## Patentansprüche

1. Schutzteil-Abnahmevorrichtung zum Abnehmen eines Schutzteils von einer Vorrichtung, mit:
einem rohrförmigen Teil (22,52,92) mit einem unteren Ende (24,54, 94) und einem oberen Ende (26,56,96), das zur Aufnahme eines Schutzteils (10) dimensioniert ist,
wobei das rohrförmige Teil (22,52,92) eine Innenwand hat, die mindestens ein von ihr nach innen abstehendes Greifelement (36,72,104) aufweist, wobei das mindestens eine Greifelement (36,72,104) in der Lage ist, sich radial nach außen zu biegen, um das Schutzteil (10) aufzunehmen,
**dadurch gekennzeichnet, dass**
das mindestens eine Greifelement (36,72,104) ferner in der Lage ist, sich nach innen zu biegen, um mit einer Rippe oder Lippe (12) des Schutzteils (10) verriegelnd zusammenzugreifen, wenn das Schutzteil (10) in das rohrförmige Teil (22,52,92) eingeführt wird;
die Schutzteil-Abnahmevorrichtung ferner aufweist:
eine von dem unteren Ende (24,54,94) des rohrförmigen Teils (22,52, 92) nach außen abstehenden Basis (28,58), die eine im Wesentlichen ebene untere Fläche (34,60) aufweist, und
eine Schutzabdeckung (62), die von dem rohrförmigen Teil (22,52,92) im Abstand von der Basis (28,58) nach außen absteht, wodurch ein Spalt (38) zwischen der Basis und der Schutzabdeckung gebildet wird;
wobei die Basis (28,58) einander gegenüberliegende Arme (30,64) aufweist, die jeweils eine obere Fläche (32,66) mit einer im Wesentlichen gekrümmten Form haben, welche eine konturierte Vertiefungsfläche zum Stabilisieren der Schutzteil-Abnahmevorrichtung bildet.

2. Vorrichtung nach Anspruch 1, bei der das rohrförmige Teil (22,52,92) mehrere von dem rohrförmigen Teil (22,52,92) nach innen abstehende Greifelemente (36,72,104) zum Umfassen des Schutzteils (10) aufweist.

3. Vorrichtung nach Anspruch 2, bei der jedes der Greifelemente (36,72, 104) eine Einkerbung (40,78,106) aufweist, die ein Biegen der Greifelemente (36,72,104) radial nach außen ermöglicht, um die Vorrichtung zu befähigen, Schutzteile (10) mit verschiedenen Bemessungen zu umfassen.

4. Vorrichtung nach Anspruch 2, bei der jedes Greifelement (36,72,104) durch eine in dem rohrförmigen Teil (22,52,92) gelegene Einkerbung (40,78,106) unterbrochen ist, die dem Greifelement ermöglicht, sich radial nach außen zu biegen und dadurch die Vorrichtung zu befähigen, Schutzteile (10) mit verschiedenen Bemessungen zu umfassen.

5. Vorrichtung nach Anspruch 1, bei der die Basis (28,58) ein Stabilisierungsteil aufweist, das an dem rohrförmigen Teil (22,52,92) radial nach außen in einer zu den einander gegenüberliegenden Armen (30,64) im Wesentlichen rechtwinkligen Orientierung absteht.

6. Schutzteil-Abnahmevorrichtung zum Abnehmen eines Schutzteils von einer Vorrichtung, mit:
einem rohrförmigen Teil (22,52,92) mit einem unteren Ende (24,54, 94) und einem oberen Ende (26,56,96), das zur Aufnahme eines Schutzteils (10) dimensioniert ist,
wobei die Schutzteil-Abnahmevorrichtung ferner ein im Wesentlichen konisches Teil mit einer im Durchmesser großen Öffnung (100) und einer im Durchmesser kleinen Öffnung (102) aufweist, das einstückig mit dem oberen Ende (26,56,96) des rohrförmigen Teils (22,52,92) ausgebildet ist;
wobei das rohrförmige Teil (52) mehrere von dem rohrförmigen Teil (52) nach innen abstehende Greifelemente (36,72,104) aufweist, wobei jedes der mehreren Greifelemente mindestens eine Einkerbung (40,78,106) aufweist, die einen Biegepunkt (42,80,108) an den Greifelementen bildet, welcher den Greifelementen ermöglicht, sich radial nach außen zu biegen, um das Schutzteil (10) aufzunehmen;
**dadurch gekennzeichnet, dass**
die mehreren Greifelemente (36,72,104) in der Lage sind, sich nach innen zu biegen, um mit einer Rippe oder Lippe (12) des Schutzteils (10) verriegelnd zusammenzugreifen, wenn das Schutzteil (10) in das rohrförmige Teil (22,52,92) eingeführt wird.

7. Schutzteil-Abnahmevorrichtung nach Anspruch 6, bei der die mehreren Greifelemente (36,72,104) mit gegenseitigem Abstand umfangsmäßig um das rohrförmige Teil (52) herum angeordnet sind.

8. Schutzteil-Abnahmevorrichtung nach Anspruch 6, bei der jede Einkerbung (40,78,106) im Wesentlichen zu dem unteren Ende (24,54,94) des rohrförmigen Teils (52) hin ausmündet.

9. Schutzteil-Abnahmevorrichtung nach Anspruch 6, bei der jede Einkerbung (40,78,106) im Wesentlichen zu dem oberen Ende (26,56,96) des rohrförmigen oder konischen Teils hin ausmündet.

10. Schutzteil-Abnahmevorrichtung nach Anspruch 6, bei der die Greifelemente (36,72,104) zwei Einkerbungen (106,107) aufweisen, die im Wesentlichen in entgegengesetzten Richtungen ausmünden.

## Revendications

1. Dispositif d'enlèvement de gaine pour enlever une gaine à partir d'un ensemble comprenant :
un élément tubulaire (22, 52, 92) ayant une extrémité inférieure (24, 54, 94) et une extrémité supérieure (26, 56, 96) dimensionnées de manière à recevoir une gaine (10) ;
l'élément tubulaire (22, 52, 92) comporte une paroi intérieure ayant au moins un élément de saisie (36, 72, 104) s'étendant vers l'intérieur à partir de celle-ci, où ledit au moins un élément de saisie (36, 72, 104) est adapté pour fléchir radialement vers l'extérieur pour recevoir la gaine (10) ;
**caractérisé en ce que**
ledit au moins un élément de saisie (36, 72, 104) est en outre adapté pour fléchir vers l'intérieur pour se verrouiller sur une nervure ou une lèvre (12) de ladite gaine (10) lorsque ladite gaine (10) est insérée dans ledit élément tubulaire (22, 52, 92) ;
le dispositif d'enlèvement de gaine comprend en outre
une base (28, 58) s'étendant vers l'extérieur à partir de ladite extrémité inférieure (24, 54, 94) dudit élément tubulaire (22, 52, 92) ayant une surface inférieure globalement plane (34, 60), et
un protecteur (62) s'étendant vers l'extérieur à partir dudit élément tubulaire (22, 52, 92) espacé de ladite base (28, 58) fournissant ainsi un espace (38) entre ladite base et ledit protecteur ;
ladite base (28, 58) comportant des bras opposés (30, 64) ayant chacun une surface supérieure (32, 66) avec une forme globalement arquée fournissant une surface creuse profilée pour stabiliser ledit dispositif d'enlèvement de gaine.

2. Dispositif selon la revendication 1, dans lequel ledit élément tubulaire (22, 52, 92) comporte une pluralité d'éléments de saisie (36, 72, 104) s'étendant vers l'intérieur dudit élément tubulaire (22, 52, 92) pour serrer la gaine (10).

3. Dispositif selon la revendication 2, dans lequel chacun desdits éléments de saisie (36, 72, 104) comporte une encoche (40, 78, 106) autorisant auxdits éléments de saisie (36, 72, 104) de fléchir radialement vers l'extérieur permettant audit dispositif de serrer des gaines (10) ayant différentes dimensions.

4. Dispositif selon la revendication 2, dans lequel chacun desdits éléments de saisie (36, 72, 104) est séparé par une encoche (40, 78, 106) disposée dans ledit élément tubulaire (22, 52, 92) autorisant à chacun desdits élément de saisie de fléchir radialement vers l'extérieur permettant audit dispositif de serrer des gaines (10) ayant différentes dimensions.

5. Dispositif selon la revendication 1, dans lequel ladite base (28, 58) comporte un élément de stabilisation faisant saillie radialement vers l'extérieur au niveau dudit élément tubulaire (22, 52, 92) dans une orientation globalement perpendiculaire audit bras opposés (30, 64).

6. Dispositif d'enlèvement de gaine pour enlever une gaine à partir d'un ensemble comprenant :
un élément tubulaire (22, 52, 92) ayant une extrémité inférieure (24, 54, 94) et une extrémité supérieure (26, 56, 96) dimensionnée de manière à recevoir une gaine (10) ;
le dispositif d'enlèvement de gaine comprend en outre un élément globalement conique ayant une ouverture de grand diamètre (100) et une ouverture de petit diamètre (102) formées d'un seul tenant avec ladite extrémité supérieure (26, 56, 96) dudit élément tubulaire (22, 52, 92) ;
dans lequel ledit élément tubulaire (52) comporte une pluralité d'éléments de saisie (36, 72, 104) s'étendant vers l'intérieur dudit élément tubulaire (52), chacun de ladite pluralité d'éléments de saisie ayant au moins une encoche (40, 78, 106) fournissant un point de flexion (42, 80, 108) sur lesdits éléments de saisie permettant auxdits éléments de saisie de fléchir radialement vers l'extérieur pour recevoir la gaine (10) ;
**caractérisé en ce que**
ladite pluralité d'éléments de saisie (36, 72, 104) sont adaptés pour fléchir vers l'intérieur pour se verrouiller sur une nervure ou une lèvre (12) de ladite gaine (10) lorsque ladite gaine (10) est insérée dans ledit élément tubulaire (22, 52, 92).

7. Dispositif d'enlèvement de gaine selon la revendication 6, dans lequel ladite pluralité d'éléments de saisie (36, 72, 104) sont espacés de manière circonférentielle autour dudit élément tubulaire (52).

8. Dispositif d'enlèvement de gaine selon la revendication 6, dans lequel chacune desdites encoches (40, 78, 106) débouche globalement vers ladite extrémité inférieure (24, 54, 94) dudit élément tubulaire (52).

9. Dispositif d'enlèvement de gaine selon la revendication 6, dans lequel ladite encoche (40, 78, 106) débouche globalement vers ladite extrémité supérieure (26, 56, 96) dudit élément tubulaire ou conique.

10. Dispositif d'enlèvement de gaine selon la revendication 6, dans lequel lesdits éléments de saisie (36, 72, 104) comportent deux encoches (106, 107) débouchant dans des directions globalement opposées.
